# EUROPEAN PATENT APPLICATION

(11) **EP 4 365 277 A1**
(43) Date of publication of application: **08.05.2024**
(21) Application number: 22832610.4
(22) Date of filing: 09.05.2022
(51) Int. Cl.: C12M 1/34, C12Q 1/02, G16B 40/00

(54) **CELL-CULTURING-RESULT PREDICTION METHOD, CULTURING-RESULT PREDICTION PROGRAM, AND CULTURING-RESULT PREDICTION DEVICE**

(30) Priority: 29.06.2021 JP 2021107843
(71) Applicant: FUJIFILM Corporation, Tokyo 106-8620 (JP)
(72) Inventor: TERAO Takahiro, Ashigarakami-gun, Kanagawa 258-8577 (JP); NAGASE Masaya, Ashigarakami-gun, Kanagawa 258-8577 (JP)
(74) Representative: Klunker IP Patentanwälte PartG mbB
(86) International application number: PCT/JP2022/019692
(87) International publication number: WO 2023/276450

(57) **Abstract**

Provided are a method for predicting a cell culture result, a culture result prediction program, and a culture result prediction apparatus that predict a culture result of an entire culture process. The method for predicting a cell culture result includes receiving a culture environment including a culture medium composition and a culture condition for culturing cells; predicting a biological behavior amount on the basis of the culture environment; changing a cell environment on the basis of the predicted biological behavior amount; repeating prediction of the biological behavior amount and change of the cell environment on the basis of the changed cell environment; and outputting the changed cell environment. Further, a culture result prediction program for executing the method for predicting a cell culture result, and a culture result prediction apparatus are provided.

## Description

### BACKGROUND OF THE INVENTION

### 1. Field of the Invention

The present invention relates to a method for predicting a cell culture result, a culture result prediction program, and a culture result prediction apparatus, and more specifically to a method for predicting a cell culture result, a culture result prediction program, and a culture result prediction apparatus for searching for culture conditions for producing bioproduction.

### 2. Description of the Related Art

In the examination of culture conditions (such as a culture medium, a culture method, and a period) for bioproduction production, efficient search for optimum conditions has been performed using a computer. In recent years, studies on the analysis of metabolic circuits have been developed for the purpose of metabolic control such as fermentation control. To search for optimum culture conditions by using a computer, it is necessary to measure, in addition to the structure of a metabolic circuit, constants related to the respective reactions, and so on. However, it is difficult to measure constants related to intracellular reactions, except for some models. As a method for examining culture conditions for bioproduction production from calculable metabolism by using partially known constants, for example, a method described in JP2007-47994A is known.

### SUMMARY OF THE INVENTION

For desired cells and bioproduction production, the consumption and production of substances whose mechanisms of action are clear can be simulated on the basis of metabolic circuit information of the cells. For growth inhibition, cell death, and suppression of antibody production for which the mechanisms of action are unclear, however, it is difficult to perform mechanism-based modeling, and the numerical values of these factors are set to constants to perform a simulation. Accordingly, it is required to also model these numerical values with high accuracy to calculate a more accurate culture result that is reflected in simulation.

The present invention has been made in view of such circumstances, and provides a method for predicting a cell culture result, a culture result prediction program, and a culture result prediction apparatus that predict a culture result without repeating an experiment.

To achieve the object of the present invention, a method for predicting a cell culture result according to the present invention includes receiving a culture environment constituted by a culture medium composition and a culture condition for culturing cells; predicting a biological behavior amount on the basis of the culture environment; changing a cell environment on the basis of the predicted biological behavior amount; repeating prediction of the biological behavior amount and change of the cell environment on the basis of the changed cell environment; and outputting the changed cell environment.

According to an aspect of the present invention, preferably, the biological behavior amount represents a change in the number of cells over time, changes in a substance produced by the cells and an amount thereof over time, and changes in a substance included in the culture environment and an amount thereof over time.

According to an aspect of the present invention, preferably, the cell environment represents a total number of the cells and a substance produced by the cells and an amount thereof.

According to an aspect of the present invention, preferably, the predicting of the biological behavior amount includes a method using a trained model determined by machine learning.

According to an aspect of the present invention, preferably, the predicting of the biological behavior amount includes a method using a trained model determined by machine learning, and a method using a biological mechanism-of-action model.

According to an aspect of the present invention, preferably, the biological mechanism-of-action model includes a modeling approach including flux balance analysis or metabolic flux analysis using a genome-scale metabolic model, or a cell signaling model represented by a differential equation.

According to an aspect of the present invention, preferably, the method using the trained model predicts cell growth inhibition, cell death, and suppression of production of a substance produced by a cell.

According to an aspect of the present invention, preferably, the cells are eukaryotic cells or prokaryotic cells.

According to an aspect of the present invention, preferably, the eukaryotic cells are animal, plant, or insect derived cell lines, primary cultures, or fungi.

According to an aspect of the present invention, preferably, the prokaryotic cells are bacteria including Escherichia coli, Bacillus subtilis, cyanobacteria, or actinomycetes, and archaebacteria including methanogens, hyperhalophiles, or hyperthermophiles.

To achieve the object of the present invention, a culture result prediction program according to the present invention causes a computer to execute the method for predicting a cell culture result described above.

To achieve the object of the present invention, a culture result prediction apparatus according to the present invention includes a processor configured to receive a culture environment constituted by a culture medium composition and a culture condition for culturing cells; predict a biological behavior amount on the basis of the culture environment; change a cell environment on the basis of the predicted biological behavior amount; repeat prediction of the biological behavior amount based on the changed cell environment and change of the cell environment based on the predicted biological behavior amount; and output the changed cell environment.

According to the present invention, it is possible to accurately predict a culture result on the basis of a culture environment without repeating an experiment.

### BRIEF DESCRIPTION OF THE DRAWINGS

Fig. 1 is a block diagram illustrating a configuration of a culture result prediction apparatus;
Fig. 2 is a block diagram illustrating a configuration of a processing unit;
Fig. 3 is a flowchart illustrating a method for predicting a cell culture result;
Fig. 4 is a diagram illustrating an overview of a simulator for a biological behavior amount prediction step;
Fig. 5 is a schematic diagram illustrating cellular metabolic pathways;
Fig. 6 is a diagram illustrating an overview of the Michaelis-Menten equation;
Fig. 7 is a diagram illustrating an overview of existing cell metabolism models;
Fig. 8 is a schematic diagram illustrating creation of a cell death model;
Fig. 9 depicts graphs illustrating the relationship between the cell concentration and the titer (antibody titer) with respect to the number of days of culture; and
Fig. 10 depicts graphs illustrating the results of an example.

### DESCRIPTION OF THE PREFERRED EMBODIMENTS

A method for predicting a cell culture result, a culture result prediction program, and a culture result prediction apparatus according to an embodiment of the present invention will be described hereinafter with reference to the accompanying drawings.

### Culture Result Prediction Apparatus

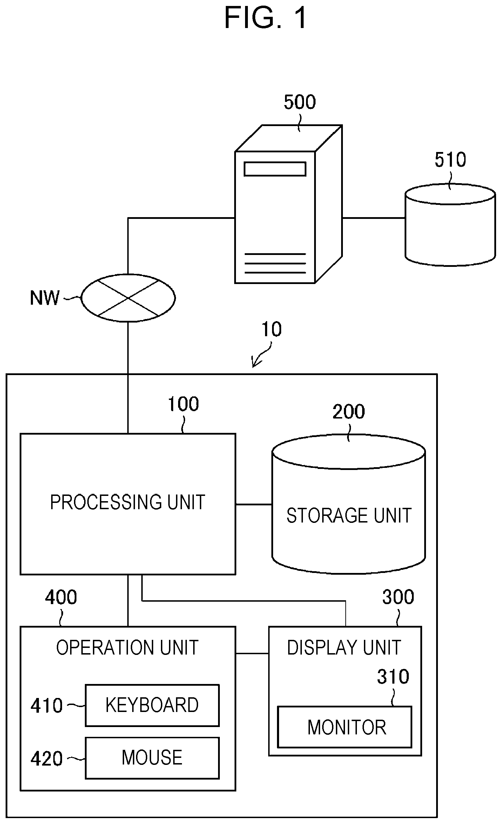

Fig. 1 is a block diagram illustrating a configuration of a culture result prediction apparatus (hereinafter also simply referred to as "prediction apparatus") 10. The prediction apparatus 10 is an apparatus that predicts a culture result on the basis of an input culture environment, and can be implemented using a computer. As illustrated in Fig. 1, the prediction apparatus 10 includes a processing unit 100, a storage unit 200, a display unit 300, and an operation unit 400, which are connected to each other to transmit and receive necessary information. These components can be installed in various ways. The components may be installed in one location (such as in one housing or one room) or may be installed in separate locations and connected to each other via a network. The prediction apparatus 10 can also be connected to an external server 500 and external data 510 via a network NW such as the Internet to acquire a culture medium composition and culture conditions or information used for simulation, such as conditions and mathematical models, as necessary.

### Configuration of Processing Unit

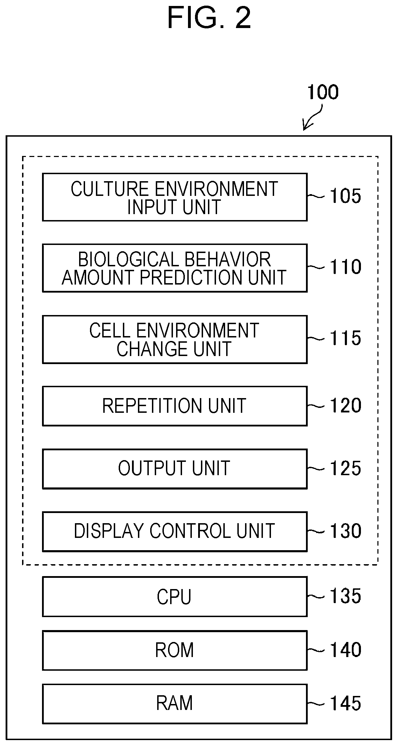

Fig. 2 is a diagram illustrating a configuration of the processing unit 100. The processing unit 100 includes a culture environment input unit 105, a biological behavior amount prediction unit 110, a cell environment change unit 115, a repetition unit 120, an output unit 125, a display control unit 130, a CPU 135 (CPU: Central Processing Unit), a ROM 140 (ROM: Read Only Memory), and a RAM 145 (RAM: Random Access Memory).

The culture environment input unit 105 receives a culture environment constituted by a culture medium composition and culture conditions for culturing cells. The biological behavior amount prediction unit 110 predicts a biological behavior amount by simulation on the basis of the culture environment input to the culture environment input unit 105. The cell environment change unit 115 changes the cell environment on the basis of the biological behavior amount predicted by the biological behavior amount prediction unit 110. The repetition unit 120 performs control to repeat the prediction of the biological behavior amount, which is performed by the biological behavior amount prediction unit 110, and the change of the cell environment, which is performed by the cell environment change unit 115, until a predetermined culture period elapses. At the repetition unit 120, the cell environment is calculated with the repetition of the prediction of the biological behavior amount and the change of the cell environment to calculate a cell environment changed by culture. The output unit 125 outputs the cell environment changed by the cell environment change unit 115. The output unit 125 can further output culture conditions input to the culture environment input unit 105. Further, the output unit 125 can also output a biological behavior amount predicted in a biological behavior amount prediction step, and results determined from them, such as the cell growth curve and the progress of antibody production. The display control unit 130 controls display of the acquired information and processing results on a monitor 310. A method for predicting a cell culture result by using these functions of the processing unit 100 will be described in detail. The processing by these functions is performed under the control of the CPU 135.

The functions of the components of the processing unit 100 described above can be implemented using various processors. The various processors include, for example, a CPU, which is a general-purpose processor that executes software (program) to implement various functions. The various processors described above also include a programmable logic device (PLD) that is a processor whose circuit configuration can be changed after manufacture, such as an FPGA (Field Programmable Gate Array). The various processors described above further include a dedicated electric circuit that is a processor having a circuit configuration designed specifically for executing specific processing, such as an ASIC (Application Specific Integrated Circuit).

The functions of the components may be implemented by a single processor or a combination of a plurality of processors. Alternatively, a plurality of functions may be implemented by a single processor. Examples of configuring a plurality of functions by a single processor include, first, a form in which, as typified by a computer such as a client or server computer, the single processor is configured by a combination of one or more CPUs and software and the processor is implemented as a plurality of functions. The examples include, second, a form in which, as typified by a system on chip (SoC) or the like, a processor is used in which the functions of the entire system are implemented by a single IC (Integrated Circuit) chip. As described above, the various functions are configured using one or more of the various processors described above as a hardware structure. More specifically, the hardware structure of the various processors is an electric circuit (circuitry) including a combination of circuit elements such as semiconductor elements.

When the processor or electric circuit described above is to execute software (program), the processor (computer) readable code of the software to be executed is stored in a non-transitory recording medium such as the ROM 140 (see Fig. 2), and the processor refers to the software. The software to be stored in the non-transitory recording medium includes a program for executing the method for predicting a cell culture result according to the embodiment of the present invention. The code may be recorded on a non-transitory recording medium such as various magneto-optical recording devices or semiconductor memories, instead of the ROM 140. In the processing using software, for example, the RAM 145 is used as a temporary storage area. For example, data stored in an EEPROM (Electronically Erasable and Programmable Read Only Memory) (not illustrated) can be referred to.

### Configuration of Storage Unit

The storage unit 200 is constituted by a non-transitory recording medium such as a DVD (Digital Versatile Disk), a hard disk, or various semiconductor memories, and a control unit therefor. The storage unit 200 stores the culture environment input to the culture environment input unit 105 and the biological behavior amount predicted by the biological behavior amount prediction unit 110. The storage unit 200 further stores the cell environment changed by the cell environment change unit 115 and results determined from them, such as the cell growth curve and the progress of antibody production.

### Configuration of Display Unit and Operation Unit

The display unit 300 includes the monitor 310 (display device) and is capable of displaying input information, information stored in the storage unit 200, a result of processing performed by the processing unit 100, and so on. The operation unit 400 includes a keyboard 410 and a mouse 420 as an input device and/or a pointing device. The user can use these devices and the screen of the monitor 310 to perform operations necessary to execute the method for predicting a cell culture result according to the present embodiment. The operations that the user can execute include input of a culture environment constituted by a culture medium composition and culture conditions for culturing cells, and so on.

### Process in Culture Result Prediction Apparatus

The prediction apparatus 10 described above can predict a cell culture result in accordance with an instruction given by the user through the operation unit 400.

### Method for Predicting Cell Culture Result

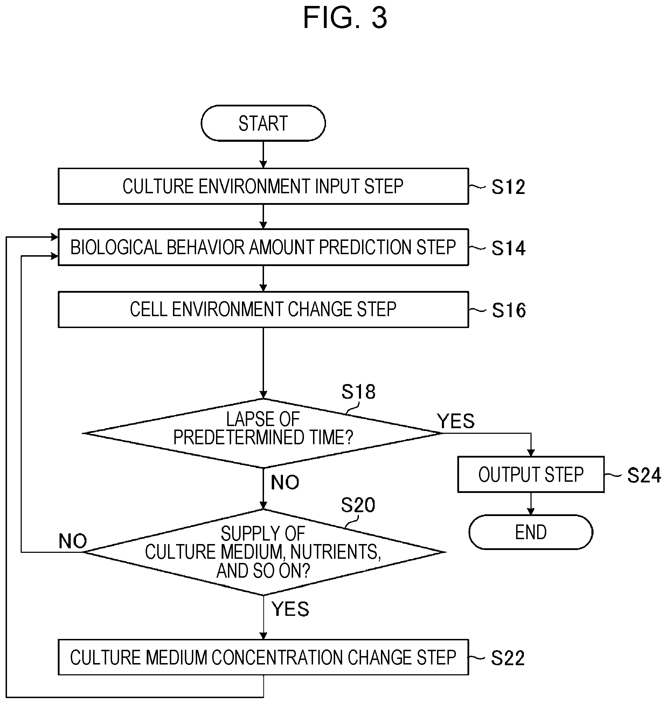

Fig. 3 is a flowchart illustrating the method for predicting a cell culture result according to the embodiment of the present invention. The flowchart illustrating the method for predicting a cell culture result according to the embodiment of the present invention has a culture environment input step of inputting a culture environment constituted by a culture medium composition and culture conditions for culturing cells, a biological behavior amount prediction step of predicting a biological behavior amount on the basis of the culture environment input in the culture environment input step, and a cell environment change step of changing a cell environment on the basis of the biological behavior amount predicted in the biological behavior amount prediction step. The flowchart further has a repetition step of repeating the biological behavior amount prediction step of predicting a biological behavior amount on the basis of the cell environment changed in the cell environment change step and the cell environment change step until a predetermined time that is a culture time elapses. The flowchart further has an output step of outputting the cell environment changed in the cell environment change step after the predetermined time has elapsed.

The steps will be described hereinafter.

### Culture Environment Input Step (Step S12)

The culture environment input unit 105 of the prediction apparatus 10 performs a culture environment input step (step S12). The culture environment input step is a step of inputting a culture environment constituted by a culture medium composition and culture conditions for culturing cells. The culture environment is input by the user and is processed by the culture environment input unit 105.

Examples of the culture medium composition include culture medium components of a culture medium for culturing the cells and the amounts of the culture medium components. The culture conditions are setting conditions for optimizing a cell culture process. Examples of the culture conditions include conditions such as a culture method, the size and type of a culture vessel, the addition of oxygen, the supply of a culture medium and a nutrient source, the addition of a pH adjuster and carbon dioxide, the removal of a culture medium containing growth-inhibiting by-products, and the harvest of a target product.

Examples of the culture method include whether to perform sterilization treatment, whether the culture medium is a liquid or solid, and the culture temperature. Further, as the culture conditions, not only the conditions at the start of culture, but also items during the culture period, such as the presence or absence and amount of oxygen added, the presence or absence and amount of a culture medium and a nutrient source supplied, the addition of a pH adjuster and carbon dioxide, the presence or absence and amount of a culture medium containing growth-inhibiting by-products that is removed, and the presence or absence and amount of a target product harvested can be set.

### Biological Behavior Amount Prediction Step (Step S14)

The biological behavior amount prediction unit 110 of the prediction apparatus 10 performs a biological behavior amount prediction step (step S14). The biological behavior amount prediction step is a step of predicting a biological behavior amount on the basis of the culture environment input in the culture environment input step. The biological behavior amount represents a change in the number of cells over time, changes in substances produced by the cells and the amount thereof over time, and changes in substances included in the culture environment and the amount thereof over time. The substances produced by the cells are antibodies and the like produced by the cells. The substances included in the culture environment are culture medium components, by-products, and the like.

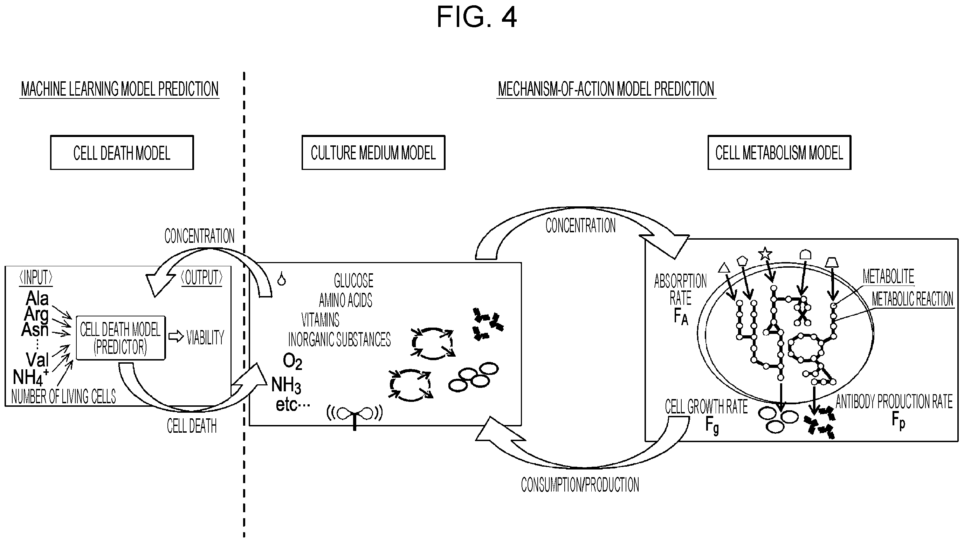

Fig. 4 is a diagram illustrating an overview of a simulator for the biological behavior amount prediction step. As illustrated in Fig. 4, the biological behavior amount prediction step is performed by using a mechanism-of-action model (biological mechanism-of-action model) and a machine learning model. As the mechanism-of-action model, respective mathematical models for the inside and outside of a cell are used. For the inside of the cell, a cell metabolism model for calculating a cell growth rate F_{g} and an antibody production rate Fₚ by using an absorption rate F_{A} at which the cell absorbs each component of the culture medium is used. For the outside of the cell, a culture medium model for calculating a change in the concentration of each component of the culture medium is used. For cell death, a model determined by using machine learning is used.

First, a cell metabolism model performed inside a cell will be described. In the cell metabolism model, the cell growth rate and the antibody production rate can be determined by using a cell culture simulation method that reproduces a bioprocess and the mechanism of a cell to be cultured. The cell culture simulation method can be performed by using a method including a modeling approach including flux balance analysis (FBA) or metabolic flux analysis (MFA) using a genome-scale metabolic model.

The "flux" or "metabolic flux" refers to the rate at which molecules pass through a target pathway or reaction. Among the factors that control flux are the rate of catalysis of enzymes in the pathway, the availability (durability) of substrate, the concentration of enzymes in the cell, and the proximity of enzymes in the pathway. The "metabolic flux analysis method" is a method for determining the amount of molecules that migrate from these factors. The "flux balance analysis" is an analysis method focusing on stoichiometry and metabolic flow rate, and, even when constants related to metabolism are not fully measurable, analyzes the behavior range of a target metabolic circuit and features thereof from the structure of a metabolic reaction on the basis of basic constraint conditions such as the law of conservation of mass.

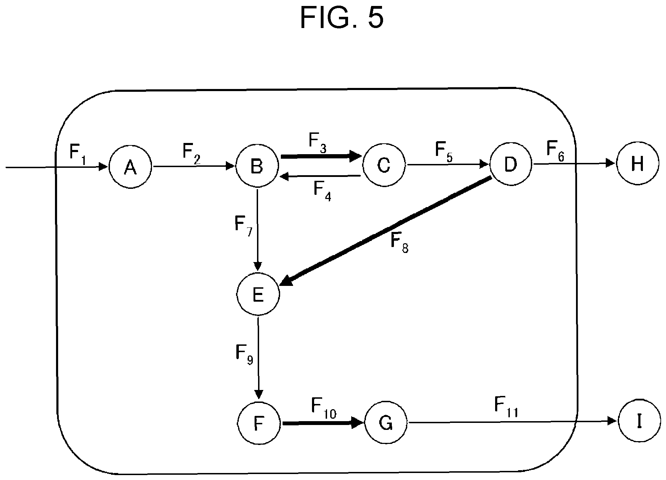

Next, intracellular metabolism will be described with reference to Fig. 5. Fig. 5 is a schematic diagram illustrating an example of cellular metabolic pathways. For a cell metabolism model, first, uptake constraint conditions, which are upper limits and lower limits for uptake of a substrate used for metabolism, nutrients, and the like in a culture medium, are acquired. In Fig. 5, substances generated by metabolism are represented by A, B, C, etc. F₁, F₂, F₃, etc. represent functions indicating the respective changes in the concentrations of the substances over time. For example, F₁ represents a flux at which the substance A is taken in, and F₂ represents a flux at which the substance B is made through metabolism.

In the example illustrated in Fig. 5, the conversion of the substance A into the substance B is performed in equal amount in accordance with the law of conservation of mass. Likewise, the amount of the substance B is equal to the total amount of conversion into the substance C and the substance E. As described above, the amount of a substance to be converted is determined by the amounts of a substrate and a nutrient to be initially taken in by cells, and so on. Another parameter that limits the conversion of a substance is the reaction rate. For example, as described above, the conversion of the substance A into the substance B is performed in equal amount. However, in a certain unit time, the conversion into the substance B has an upper limit due to the limited rate of reaction, and not all the substance A may be converted into the substance B. This parameter for limiting the conversion of a substance is acquired as an uptake constraint condition, and the uptake constraint condition is acquired by using an expression obtained by mathematically modeling a mechanism by which a cell takes in a culture medium component, and is applied to calculation of a culture result.

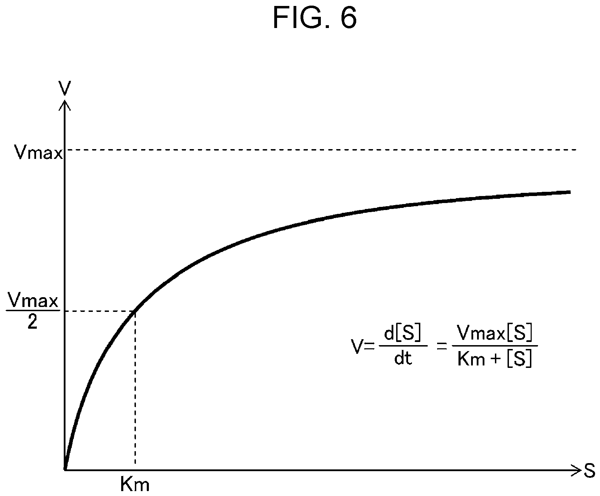

As a mathematical model for determining the reaction rate, for example, the Michaelis-Menten equation can be used. Fig. 6 illustrates an overview of the Michaelis-Menten equation. The Michaelis-Menten equation is an equation related to a reaction rate V of an enzyme. When a substrate concentration S is low, the reaction rate V is proportional to the substrate concentration S. When the substrate concentration S is high, the reaction rate V converges to a maximum rate Vmax regardless of the substrate concentration S. Other mathematical models that can be used include Fick's law. Fick's law is an expression used to determine a flux that is the amount of substance passing through a unit area per unit time, and the flux is proportional to a diffusion coefficient D and a substrate concentration gradient on both sides of a membrane. In the present embodiment, the Michaelis-Menten equation or Fick's law is used as a constraint condition for taking in a culture medium. This makes it possible to perform a simulation without calculating a condition in which a reaction rate is higher than the reaction rate and the flux determined by the mathematical models but falls within the range of the amount of conversion determined by the law of conservation of mass. Accordingly, more accurate culture prediction can be performed.

The calculation by simulation may be performed by using, in parallel, a plurality of mathematical models that mimic the state of a cell. For example, a mathematical model for cell growth and a mathematical model for production of only bioproduction without cell growth can be arranged in parallel, and the ratio between the plurality of models can be changed in accordance with the culture state such as the culture medium concentration.

Next, a culture medium model performed outside a cell will be described. The culture medium model is a model for determining a change in the concentration of a culture medium surrounding a cell when metabolism occurs under the conditions of the cell metabolism model described above, and a cell signaling model represented by a differential equation can be used. The concentration change of each component can be determined by solving an ordinary differential equation for time t using the Runge-Kutta method.

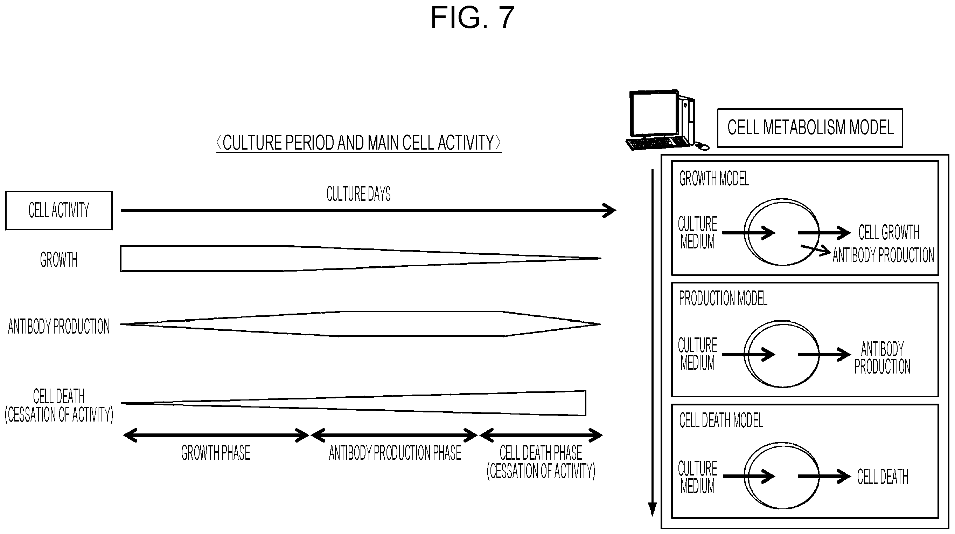

Existing cell metabolism models including a cell death model will now be described. Fig. 7 is a diagram illustrating an overview of existing cell metabolism models. The main cell culture activity during the culture period is divided into cell growth, antibody production by cells, and cell death (cessation of activity). As illustrated in Fig. 7, as the phases of these activities, the early stage of culture has a growth phase in which cells grow, and the middle stage of culture has an antibody production phase in which the cell growth rate decreases and the antibody production rate increases. Subsequently, the late stage of culture has a cell death phase in which cell death, which is the cessation of cellular activity, occurs. In an existing simulation for culture prediction, these three culture activities are performed by using a cell metabolism model, and calculation is performed by using a growth model for cell growth, a production model for antibody production, and a cell death model for cell death. However, the cell death model is implemented as a simple model with constants, and sufficient accuracy is not obtained. In the present embodiment, accordingly, as illustrated in Fig. 4, a trained model obtained by machine learning is used as a cell death model to perform a simulation to calculate a prediction result of culture with high accuracy.

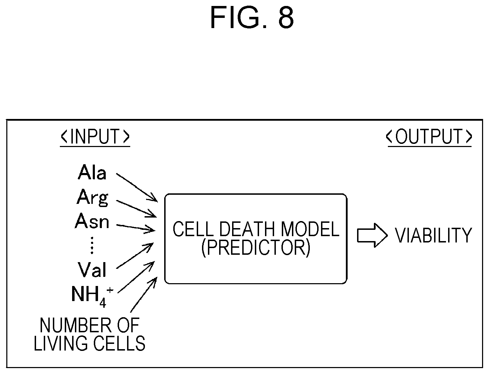

Fig. 8 is a schematic diagram illustrating creation of a cell death model by using machine learning. A random forest method can be used as a method for creating a trained model by machine learning. When a cell death model is constructed by machine learning, the number of living cells, the concentrations of 20 types of amino acids, and the concentration of ammonia are input as data to the input side. The cell viability is input as data to the output side. As the data to be used, 80% of the data obtained by actual culture and measurement is used to perform learning, and 20% thereof is used to perform a test. This makes it possible to create a cell death model. The proportion of the measurement data used for learning and the proportion of the measurement data used for testing can be set as appropriate.

The creation of a trained model by machine learning is not limited to that for a cell death model. For a data item that is difficult to model based on a mechanism, such as cell death, a trained model is created by machine learning to calculate a prediction result of culture with higher accuracy. Examples of such a data item include growth inhibition and suppression of antibody production. To create a growth inhibition model by machine learning, the amount of cell growth (growth ratio) can be used as data to be used for the output side. To create an antibody production suppression model, the amount of antibody production (production ratio) can be used as data to be used for the output side.

In the biological behavior amount prediction step according to the present embodiment, a trained model created by machine learning is used for cell death, thereby making it possible to measure a biological behavior amount with higher accuracy. As a result, a prediction result of culture can be calculated with high accuracy.

The biological behavior amount prediction step (step S14) can be performed on the basis of the culture conditions (concentrations of culture medium components) and the uptake constraint conditions acquired in the culture condition input step (step S12), by using a CHO cell simulator including these models.

### Cell Environment Change Step (Step S16)

The cell environment change unit 115 of the prediction apparatus 10 performs a cell environment change step (step S16). The cell environment change step changes a cell environment on the basis of the biological behavior amount predicted in the biological behavior amount prediction step. The cell environment represents the total number of cells, substances produced by the cells, and the amounts of the substances.

Through the biological behavior amount prediction step, the change in the number of cells over time, the changes in substances produced by the cells and the amount thereof over time, and the changes in substances included in the culture environment and the amount thereof over time, described above, after a culture time t has elapsed are determined by calculation. In the cell environment change step, the changes in the number of cells and the components of the culture medium after the culture time t has elapsed are reflected in the cell environment.

### Repetition Step

The repetition unit 120 of the prediction apparatus 10 controls implementation of a repetition step. The repetition step is a step of repeating the biological behavior amount prediction step (step S14) and the cell environment change step (step S16).

After the completion of the cell environment change step (step S16), the repetition unit 120 determines whether a predetermined culture time has elapsed (step S18). If the predetermined culture time (for example, 14 days) for the calculation has elapsed (if YSE is determined), the calculation ends, and the process proceeds to the output step (step S24). If the predetermined culture time has not elapsed (if NO is determined), it is determined whether to supply a culture medium, nutrients, and so on (step S20). If no culture medium, nutrients, or the like is to be supplied (if NO is determined), the process returns to the biological behavior amount prediction step (step S14). If a culture medium, nutrients, and the like are to be supplied (if YES is determined), a culture medium concentration change step (step S22) is performed.

If the supply of a culture medium, nutrients, and the like after a predetermined time has elapsed is set as a culture condition in the culture environment input step (step S12), the culture medium concentration change step (step S22) is performed. The supply of a culture medium, nutrients, and the like also includes the supply of a pH adjuster and carbon dioxide in addition to the culture medium and the nutrients. The culture medium concentration change step adds the supplied culture medium and nutrients to the cell environment at the point in time when the cell environment change step is completed to change the culture medium concentration.

The culture medium concentration change step can involve not only supplying a culture medium, nutrients, and so on but also removing a culture medium containing growth-inhibiting by-products and changing the culture medium concentration in accordance with the harvest of a target product. If the removal of a culture medium containing growth-inhibiting by-products and the harvest of a target product are set as culture conditions in the culture environment input step (step S12), the removed growth-inhibiting by-products and the harvested target product are removed from the cell environment at the point in time when the cell environment change step is completed to change the culture medium concentration.

In the repetition step, an uptake constraint condition is acquired on the basis of the cell environment changed in the cell environment change step (step S16) or the culture medium concentration changed in the culture medium concentration change step (step S22), and the biological behavior amount prediction step (step S14) and the cell environment change step (step S16) are performed. Thereafter, the biological behavior amount prediction step (step S14) and the cell environment change step (step S16) are repeated until the predetermined culture time elapses (step S18).

After the predetermined culture period has elapsed (YES in step S18), the calculation ends. As a result, the number of cells during the culture period and the amount of generation of bioproduction, ammonia, or the like to be generated can be determined. Further, the biological behavior amounts at the respective points in time when the repetition step is performed are plotted, thereby making it possible to determine the cell growth curve, the progress of antibody production, and so on.

### Output Step (Step S24)

The output unit 125 of the prediction apparatus 10 performs an output step (step S24). The output step is a step of outputting the cell environment changed in the cell environment change step (step S16).

The output step can output, as results, the number of cells growing during the culture period and the amount of a bioproduction to be generated or the amount of generation of a bioproduction such as an antibody like ammonia. In addition, the cell growth curve, the progress of antibody production, and the like can be output. As the result to be output, the results described above after the lapse of the predetermined time may be output, or the results described above after the lapse of any period during the culture may be output.

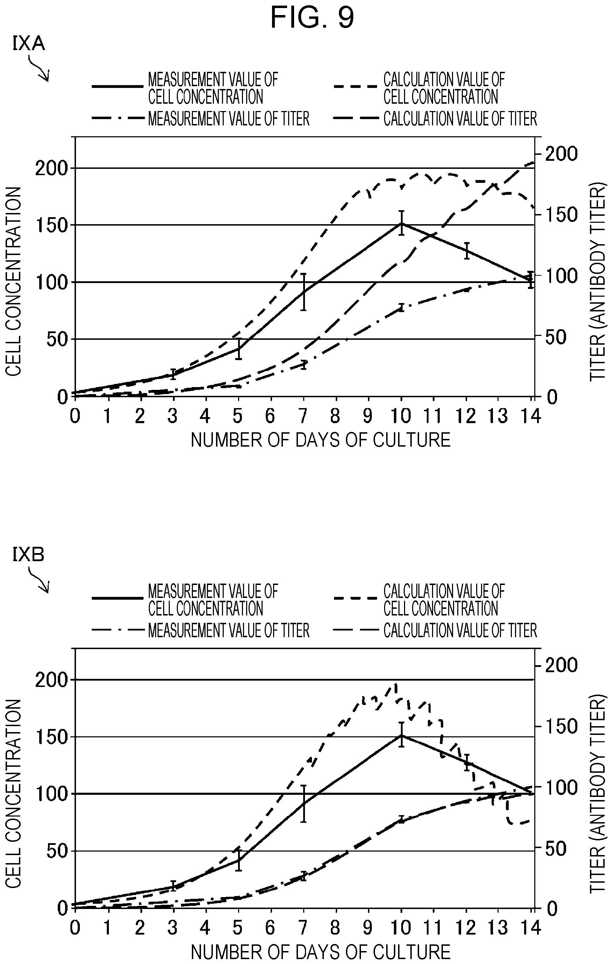

Fig. 9 depicts graphs illustrating the relationship between the cell concentration and the titer (antibody titer) with respect to the number of days of culture. A graph IXA is a graph illustrating results obtained when a trained model obtained by machine learning is not included. A graph IXB is a graph indicating results obtained when a trained model obtained by machine learning is included (the present embodiment). On the Y axis of the graphs IXA and IXB, the measurement value of the cell concentration and the measurement value of the titer (antibody titer) on day 14 of culture are set to 100. As a result of comparison between the graph IXA and the graph IXB, it can be found that simulation results close to the measurement values are obtained for the graph IXB including the trained model.

According to the method for predicting a cell culture result, the culture result prediction program, and the culture result prediction apparatus according to the embodiment of the present invention, it is possible to accurately calculate, by simulation, biological behavior amounts including growth inhibition, cell death, and production suppression for which the mechanisms of action are unclear. Thus, it is possible to accurately predict a result of cell culture of an entire culture process without repeating experiments.

### Cells Used for Simulation

The cells used in the method for predicting a cell culture result, the culture result prediction program, and the culture result prediction apparatus of the present embodiment are not specifically limited, and any of eukaryotic cells and prokaryotic cells can be used. Examples of the eukaryotic cells that can be used include animal, plant, or insect derived cell lines, primary cultures, and fungi. Examples of the prokaryotic cells that can be used include bacteria including Escherichia coli, Bacillus subtilis, cyanobacteria, and actinomycetes, and archaebacteria including methanogens, hyperhalophiles, and hyperthermophiles.

### EXAMPLE

The following provides more specific description with reference to EXAMPLE.

A simulation was performed to reproduce culture of CHO cell lines in a computer.

An experiment in which CHO cells producing antibodies were cultured was conducted to acquire time-series culture data. The CHO cell lines were cultured for 14 days, of which from day 2 to day 13, HyClone Cell Boost 7a/b (cytiva) was added once daily to perform culture. The culture data was obtained by measuring the amounts of glucose and 20 types of amino acids, the number of cells, the cell viability, the amount of generated antibodies, the amount of ammonia, and the amount of lactic acid at each of time-series measurement points on days 0, 3, 5, 7, 10, 12, and 14 for the 14 days of culture.

Machine learning was performed from the measurement data to create a trained model for inferring the cell viability. The machine learning was performed using the random forest method. Similar experiments were performed a plurality of times, and data at a total of 500 points in time, which is the sum of the results of the experiments, was used as data to be used for learning. Of the measured data, 80% was used to perform learning, and 20% was used to perform a test. As a result, a trained model for inferring the cell viability with satisfactory accuracy was created.

The metabolic circuit information and models of the CHO cells were created from FBA models (iCHOv1_final.xml) obtained from BiGG Models (http://BiGG.ucsd.edu/)) and gene-expression information measured during culture of the cell lines.

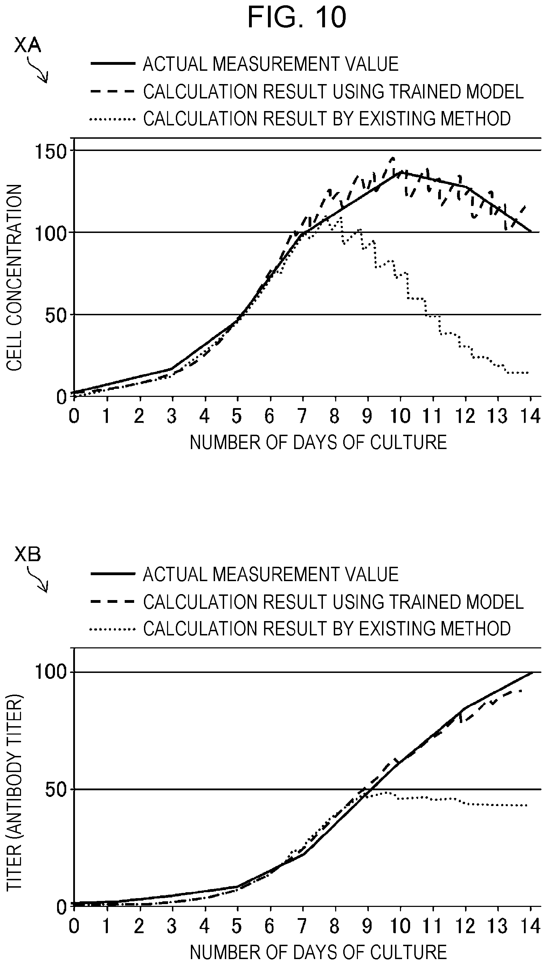

Dynamic metabolic balance analysis (dynamic FBA) was performed using an FBA model. In this operation, the prediction of cell death was performed by using a known method for setting constants and a method using a trained model. The results are illustrated in Fig. 10. In Fig. 10, a graph XAis a graph illustrating a relationship between the number of days of culture and the cell concentration, and a graph XB is a graph illustrating a relationship between the number of days of culture and the titer (antibody titer). On the Y axis of the graph XA, the actual measurement value of the cell concentration on day 14 of culture is set to 100. On the Y axis of the graph XB, the actual measurement value of the titer (antibody titer) is set to 100. As illustrated in Fig. 10, while the known method provides poor accuracy of reproduction of culture, the method using a trained model provides simulation results that well reproduce actually measured culture data.

### Reference Signs List

- 10: culture result prediction apparatus
- 100: processing unit
- 105: culture environment input unit
- 110: biological behavior amount prediction unit
- 115: cell environment change unit
- 120: repetition unit
- 125: output unit
- 130: display control unit
- 135: CPU
- 140: ROM
- 145: RAM
- 200: storage unit
- 300: display unit
- 310: monitor
- 400: operation unit
- 410: keyboard
- 420: mouse
- 500: external server
- 510: external data
- NW: network

## Claims

1. A method for predicting a cell culture result, comprising:
receiving a culture environment including a culture medium composition and a culture condition for culturing cells;
predicting a biological behavior amount on the basis of the culture environment;
changing a cell environment on the basis of the predicted biological behavior amount;
repeating prediction of the biological behavior amount and change of the cell environment on the basis of the changed cell environment; and
outputting the changed cell environment.

2. The method for predicting a cell culture result according to claim 1, wherein
the biological behavior amount represents a change in the number of cells over time, changes in a substance produced by the cells and an amount thereof over time, and changes in a substance included in the culture environment and an amount thereof over time.

3. The method for predicting a cell culture result according to claim 1 or 2, wherein the cell environment represents a total number of the cells and a substance produced by the cells and an amount thereof.

4. The method for predicting a cell culture result according to any one of claims 1 to 3, wherein
the predicting of the biological behavior amount includes a method using a trained model determined by machine learning.

5. The method for predicting a cell culture result according to any one of claims 1 to 4, wherein
the predicting of the biological behavior amount includes a method using a trained model determined by machine learning, and a method using a biological mechanism-of-action model.

6. The method for predicting a cell culture result according to claim 5, wherein
the biological mechanism-of-action model includes a modeling approach including flux balance analysis or metabolic flux analysis using a genome-scale metabolic model, or a cell signaling model represented by a differential equation.

7. The method for predicting a cell culture result according to any one of claims 4 to 6, wherein
the method using the trained model predicts cell growth inhibition, cell death, and suppression of production of a substance produced by a cell.

8. The method for predicting a cell culture result according to any one of claims 1 to 7, wherein
the cells are eukaryotic cells or prokaryotic cells.

9. The method for predicting a cell culture result according to claim 8, wherein
the eukaryotic cells are animal, plant, or insect derived cell lines, primary cultures, or fungi.

10. The method for predicting a cell culture result according to claim 8, wherein
the prokaryotic cells are bacteria including Escherichia coli, Bacillus subtilis, cyanobacteria, or actinomycetes, and archaebacteria including methanogens, hyperhalophiles, or hyperthermophiles.

11. A culture result prediction program for causing a computer to execute the method for predicting a cell culture result according to any one of claims 1 to 10.

12. A culture result prediction apparatus comprising a processor configured to:
receive a culture environment including a culture medium composition and a culture condition for culturing cells;
predict a biological behavior amount on the basis of the culture environment;
change a cell environment on the basis of the predicted biological behavior amount;
repeat prediction of the biological behavior amount based on the changed cell environment and change of the cell environment based on the predicted biological behavior amount; and
output the changed cell environment.
